# EUROPEAN PATENT SPECIFICATION

(11) **EP 3 154 454 B1**
(45) Date of publication and mention of the grant of the patent: **05.08.2020**
(21) Application number: 15809688.3
(22) Date of filing: 16.06.2015
(51) Int. Cl.: A61B 17/22, A61B 17/3207, A61B 17/3209

(54) **INTRAVASCULAR CATHETER HAVING AN EXPANDABLE INCISING PORTION AND DRUG DELIVERY MECHANISM**
INTRAVASKULÄRER KATHETER MIT EINEM EXPANDIERBAREN INZISIONSTEIL UND WIRKSTOFFFREISETZUNGSMECHANISMUS
CATHÉTER INTRAVASCULAIRE COMPORTANT UNE PARTIE D'INCISION EXPANSIBLE ET UN MÉCANISME D'ADMINISTRATION DE MÉDICAMENT

(30) Priority: 16.06.2014 US 201462012431 P
(43) Date of publication of application: 19.04.2017
(73) Proprietor: Pigott, John, P., Sylvania, OH 43560 (US)
(72) Inventor: Pigott, John, P., Sylvania, OH 43560 (US)
(74) Representative: Haseltine Lake Kempner LLP
(86) International application number: PCT/US2015/035934
(87) International publication number: WO 2015/195606

(56) References cited:
- WO-A2-02/078511
- US-A1- 2004 098 014
- US-A1- 2009 204 068
- US-A1- 2011 160 645
- US-A1- 2012 150 142
- US-A1- 2013 066 346
- US-A1- 2013 066 346
- US-A1- 2013 131 594
- US-B1- 6 283 947

## Description

### CROSS-REFERENCE TO RELATED APPLICATIONS

This application claims the benefit of U.S. Provisional Application No. 62/012,431, filed June 16, 2014.

### TECHNICAL FIELD

Embodiments of the present invention generally relate to medical devices, more specifically to intravascular catheter devices.

### BACKGROUND

Intravascular catheter devices can be used in minimally invasive surgical procedures, such as for treatment of atherosclerosis. Atherosclerosis is a chronic condition in which atheromatous plaque accumulates on the inner walls of a blood vessel. As a result, the blood vessel walls can become inflamed and, over time, may harden to form atherosclerotic lesions that cause a narrowing of the vessel lumen. In severe cases, the atherosclerotic lesions can rupture and induce the formation of thrombus (i.e., blood clots), which can prevent blood flow through the narrowed vessel lumen.

There are known procedures and devices for treating or otherwise reducing the risks associated with atherosclerosis. For example, an angioplasty is a procedure in which a balloon catheter is inserted into a narrowed region of the vessel lumen via a delivery catheter. The balloon catheter includes a flexible tube having an inflatable balloon at an end thereof. Once positioned in the narrowed region, the balloon is inflated in order to dilate the narrowed vessel lumen. The pressure in the balloon is generally sufficient to compress the accumulated plaque. However, in some cases it would be desirable to fragment the atherosclerotic lesions. Thus, it would be desirable to provide an intravascular catheter having an expandable portion that can be selectively controlled by a user and adapted to create incisions in atherosclerotic material to facilitate fragmentation of the material during an angioplasty procedure.

Further, treatment of atherosclerotic material can be improved by the use of certain drugs such as antiproliferative agents, immunosuppressive agents, angiopeptin, corticosteroids, prohelaing agents, and the like, at the site of stenosis. Therefore, it would be desirable to provide the aforementioned intravascular catheter device having a drug delivery system capable of delivering drugs at the site of the atherosclerotic plaque accumulation.

US2013/066346 (Piggott John P) discloses an intravascular catheter device is provided for use during a surgical procedure. The catheter device includes a catheter tube having an expandable portion with a plurality of struts each defining an outer surface. The expandable portion is operable between a closed position, wherein the expandable portion has a first diameter, and an opened position, wherein the expandable portion has a second diameter that is larger than the first diameter. An incising element is provided on the outer surface of at least one of the struts. The incising element has a blade that extends outwardly in a radial direction from the outer surface of the strut for creating an incision in atherosclerotic material located within a blood vessel when the expandable portion is in the opened position.

### SUMMARY OF THE INVENTIVE CONCEPT

This invention relates to an intravascular catheter device as set out in claim 1. Preferred embodiments are defined in the dependent claims.

### BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical or equivalent features, and wherein:
**FIGURE 1** is a plan view of a catheter device that includes a handle assembly and a catheter tube having an expandable incising portion, in accordance with an arrangement and indicating section line 2-2 and section line 3-3;
**FIGURE 2** is a cross-sectional side view of the handle assembly taken along section line 2-2 shown in **FIGURE 1** illustrating the catheter device is in a first operating mode;
**FIGURE 3** is an enlarged cross-sectional side view of the catheter tube taken along section line 3-3 shown in **FIGURE 1** illustrating the expandable incising portion in a first operating mode and disposed within a blood vessel, additionally indicating section line 4-4;
**FIGURE 4** is a cross-sectional end view of the expandable incising portion taken along section line 4-4 shown in **FIGURE 3****;**
**FIGURE 5** is a cross-sectional side view of the handle assembly taken along section line 2-2 shown in **FIGURE 1** illustrating the catheter device is in a second operating mode;
**FIGURE 6** is an enlarged cross-sectional side view of the catheter tube taken along section line 3-3 shown in **FIGURE 1** illustrating the expandable incising portion in the second operating mode and disposed within a blood vessel, additionally indicating section line 7-7;
**FIGURE 7** is a cross-sectional end view of the expandable incising portion taken along section line 7-7 shown in **FIGURE 6****;**
**FIGURE 8** is an enlarged side view of a catheter tube having an expandable incising portion, in accordance with another arrangement;
**FIGURE 9** is a side view of the catheter tube shown in **FIGURE 8** illustrating the expandable incising portion in an opened position and indicating section line 10-10;
**FIGURE 10** is a cross-sectional end view of the expandable incising portion taken along section line 10-10 shown in **FIGURE 9****;**
**FIGURE 11** is an enlarged side view of a catheter tube having an expandable incising portion, in accordance with an exemplary embodiment of the present invention;
**FIGURE 12** is a side view of the catheter tube shown in **FIGURE 11** illustrating the expandable incising portion in the opened position;
**FIGURE 13** is an end view of the catheter tube as shown in **FIGURE 12****;**
**FIGURE 14** is an enlarged side view of a catheter tube having an expandable incising portion, in accordance with an exemplary embodiment of the present invention;
**FIGURE 15** is a side view of the catheter tube shown in **FIGURE 14** illustrating the expandable incising portion in the opened position;
**FIGURE 16** is an end view of the catheter tube as shown in **FIGURE 15****;**
**FIGURE 17** is an enlarged cross-sectional side view similar to **FIGURE 3** illustrating another exemplary embodiment of the present invention, wherein a mechanism is provided for delivering a drug to a base of one of the incising elements;
**FIGURE 18** is an enlarged cross-sectional side view similar to **FIGURE 17** illustrating another exemplary embodiment of the present invention, wherein a mechanism is provided for delivering a drug to a surface of one of the incising elements;
**FIGURE 19** is an enlarged cross-sectional side view similar to **FIGURE 17** Illustrating another arrangement, wherein a mechanism is provided for delivering a drug to a surface of one of the incising elements; and
**FIGURE** 20 is an enlarged cross-sectional side view similar to **FIGURE 17** illustrating another arrangement, wherein one of the incising elements has a coating of medicine provided thereon.

### DETAILED DESCRIPTION OF EXEMPLARY EMBODIMENT(S)

The invention is described more fully hereinafter with reference to the accompanying drawings, in which exemplary embodiments of the invention are shown. This invention may, however, be embodied in many different forms and should not be construed as limited to the exemplary embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/ or components, but do not preclude the presence or addition of one or more other features, integers, steps, operations, elements, components, and/or groups thereof.

Embodiments of the invention are described herein with reference to illustrations that are schematic illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

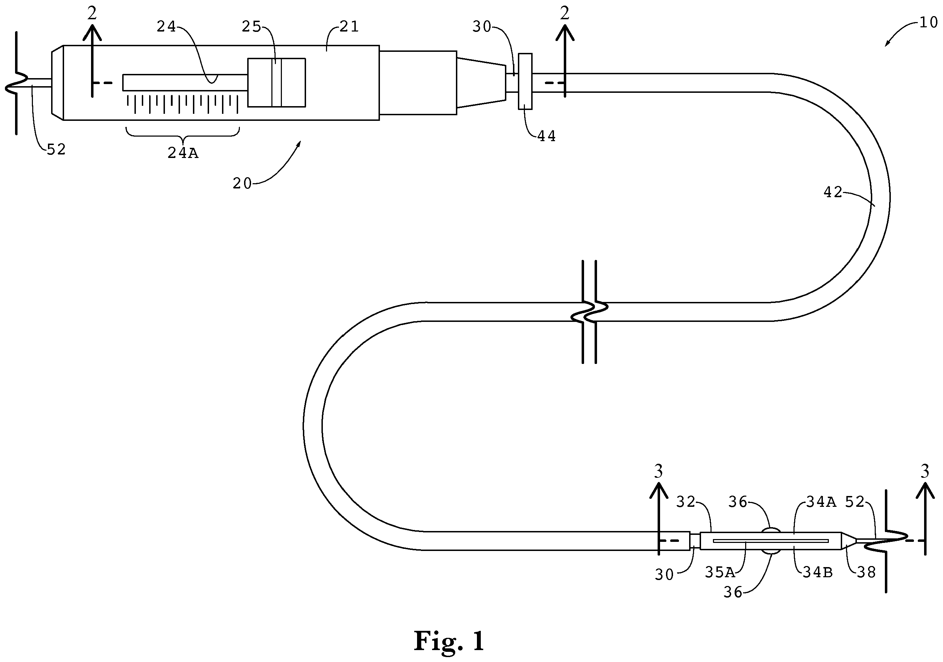

**FIGURE 1** illustrates a catheter device, indicated generally at **10.** In general, the catheter device **10** includes an expandable incising portion that can be inserted into a blood vessel and expanded to create incisions in atherosclerotic material that has accumulated on inner walls of the blood vessel. The incisions facilitate the fragmentation or compression of the atherosclerotic material during a subsequent angioplasty or atherectomy procedure. Although the catheter device **10** will be described and illustrated in the context of treating atherosclerosis, it should be appreciated that the catheter device **10** can be used in any desired environment and for any desired purpose.

The illustrated catheter device **10** includes a handle assembly, indicated generally at **20.** The illustrated handle assembly **20** includes an elongated, cylindrical handle body **21.** The handle body **21** may alternatively have any other shape that is suitable for easy handling by a surgeon. Further, the handle body **21** can be made from any suitably rigid material including, but not limited to, stainless steel or polymers.

The illustrated catheter device **10** also includes a catheter tube **30** that extends from the handle assembly **20.** The catheter tube **30** is an elongated, flexible member having a proximal end that is secured to the handle assembly **20** and a distal end that extends therefrom. The catheter tube **30** can be made from any biocompatible material including, but not limited to, polyvinyl, polyethylene, nitinol, or stainless steel. Further, the catheter tube **30** can have any outer diameter, length, or wall thickness.

An expandable portion **32** is provided on the distal end of the catheter tube **30.** The illustrated expandable portion **32** is a cylindrical member having a longitudinal axis. The expandable portion **32** can be made from a generally resilient material that is able to flex between various positions, such as polyvinyl, polyethylene, nitinol, or stainless steel. The expandable portion **32** can be secured to the catheter tube **30** in any manner including, but not limited to, a fused connection, an adhesive, a press-fit connection, a threaded connection, or any other securing means. Alternatively, the expandable portion **32** can be integrally formed from the catheter tube **30.** Further, the expandable portion **32** can have any outer diameter, length, or wall thickness.

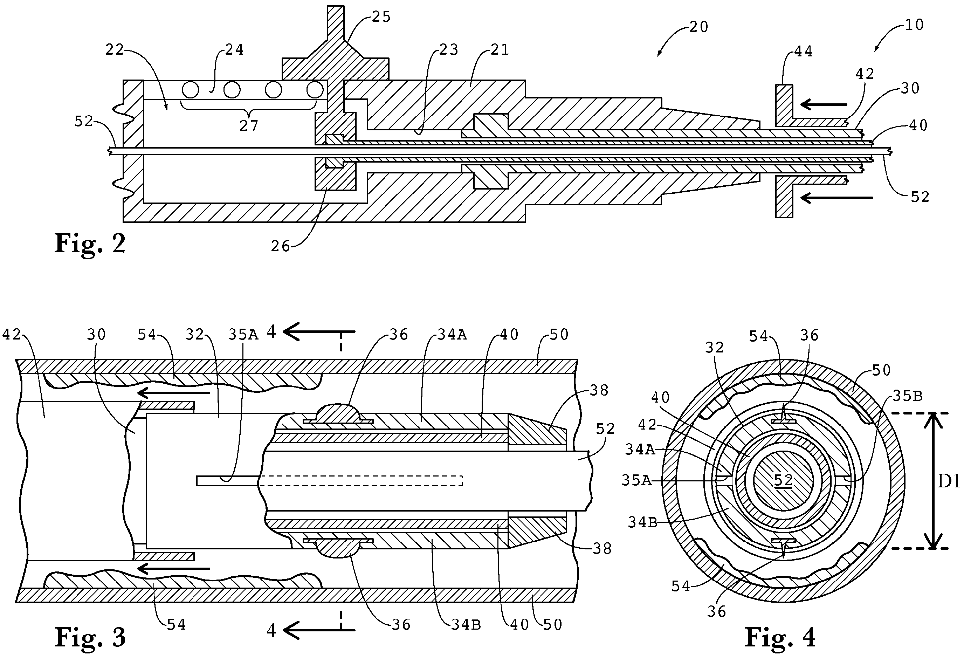

**The** illustrated expandable portion **32** has a pair of struts **34A** and **348.** The illustrated struts **34A** and **348** are separated by a pair of longitudinally extending slits **35A** and **358** that extend through side walls of the expandable portion **32.** As shown in **FIGURE 4****,** the slits **35A** and **358** are equally spaced apart from one another around the circumference of the expandable portion **32** such that the struts **34A** and **348** have the same circumferential widths, although such is not required. The struts **34A** and **348** may have any length, circumferential width, or cross-sectional shape as desired.

**The** illustrated expandable portion **32** also includes a pair of incising elements **36** that are respectively provided along outer surfaces of the struts **34A** and **348.** The incising elements **36** can be atherotomes or other incising members having arcuate shaped sharpened edges, for example, that are configured to create incisions in atherosclerotic material as will be explained below. The illustrated incising elements **36** extend parallel with the longitudinal axis of the expandable portion **32** and outwardly in a radial direction therefrom. As shown in **FIGURE 4****,** the incising elements **36** are equally spaced apart from one another around the circumference of the expandable portion **32.** The expandable portion **32** may, however, have any number or configuration of incising elements **36** provided around the circumference thereof. Further, the incising elements **36** can have any cross-sectional shape, longitudinal length, or height and can be made from any suitable material including, but not limited to, tempered steel, stainless steel, high carbon steel, or ceramics. The incising elements **36** can be molded with the struts **34A** and **348** or may otherwise be secured thereto in any manner such as, for example, using a welded or soldered connection, an adhesive, or any other fastening means.

The distal end of the expandable portion **32** may optionally include a tip member **38.** The illustrated tip member **38** has a generally conical shape that facilitates insertion of the catheter tube **30** within a blood vessel **50** (see **FIGURE 3** and **FIGURE 4**) and subsequent travel therethrough. The tip member **38** may, however, have any desired shape. An aperture may axially extend through the tip member **38,** the purpose of which will be explained below. The tip member **38** can be integrally formed with the expandable portion **32** or may be secured thereto, such as with an adhesive or the like. Further, the tip member **38** can be made from any biocompatible material including, but not limited to, polyvinyl, polyethylene, nitinol, stainless steel, or polyether block amide.

The illustrated catheter device **10** also includes a protective sheath **42** that is supported for sliding movement along an outer surface of the catheter tube **30,** although such is not required. The protective sheath **42** can be made from any biocompatible material including, but not limited to, polyvinyl, polyethylene, nitinol, or stainless steel. Further, the protective sheath **42** can have any outer diameter, length, or wall thickness.

The illustrated protective sheath **42** includes a flange **44** that facilitates sliding movement of the protective sheath **42** relative to the catheter tube **30.** The illustrated flange **44** is an annular member that is located at an end of the protective sheath **42** nearest the handle assembly **20.** The flange **44** can be integrally formed with the protective sheath **42** or may otherwise be secured thereto in any manner, such as with an adhesive or the like. It should be appreciated that the flange **44** can have any shape or may alternatively be configured in any manner to accomplish the functions described herein and below.

**FIGURE 2** and **FIGURE 3** illustrates that the handle body **21** defines an internal chamber **22.** A passage **23** extends into an end portion of the handle body **21** for communication with the internal chamber **22.** The handle body **21** further includes a slot **24** that extends through a side wall thereof for communication with the internal chamber **22.** The illustrated slot **24** may have any length or width as desired. As shown in **FIGURE 1****,** an indicator **24A** may be provided on the handle body **21** adjacent to the slot **24.** For example, the indicator **24A** can be a visual scale or any other indicating means, the purpose of which will be explained below.

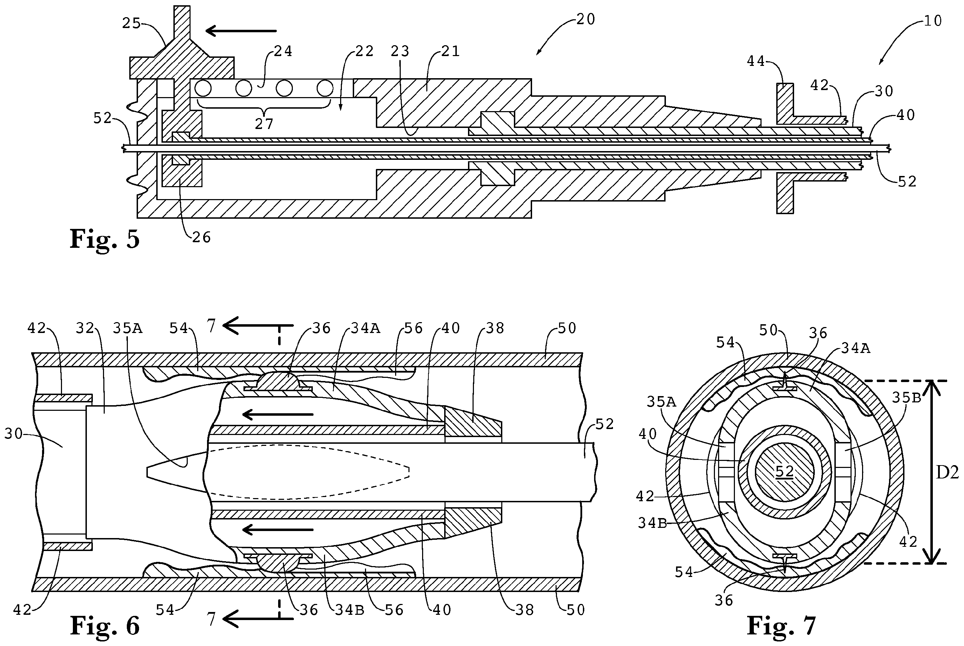

The illustrated handle assembly **20** also includes a control member **25** that is supported on the handle body **21** for sliding movement within the slot **24.** For example, the control member **25** is movable between a forward position, as illustrated in the present figure, a rearward position (shown in **FIGURE 5**), or any position therebetween, which will be further explained below. The illustrated control member **25** includes a base portion **26** that is disposed within the internal chamber **22** of the handle body **21.** The base portion **26** may define an outer cross-sectional shape that generally corresponds with a cross-sectional shape of the internal chamber **22,** although such is not required. Alternatively (or in addition), the control member **25** may be movably supported on the handle body **21** by a bearing, a bushing, a guide rail, or any other structural means. In other embodiments, the control member **25** may be supported for rotational movement, pivotal movement, or any other type of movement relative to the handle body **21,** the purpose of which will become apparent below. The visual indicator **24A,** described above, is configured to identify the relative position of the control member **25** with respect to the handle body **21.**

The illustrated handle assembly **20** also includes a locking mechanism **27** that is configured to temporarily secure the control member **25** in a desired position, although such is not required. The illustrated locking mechanism **27** includes a plurality of protrusions that are spaced apart from one another along the inner surface of the slot **24.** The control member **25** frictionally engages the protrusions to hold the control member **25** in the desired position. Alternatively, the locking mechanism **27** may be a threaded fastener, a pivotal latch, a pushbutton release, or any other mechanism that is configured to secure the control member **25** in a desired position.

The proximal end of the catheter tube **30** is secured to the handle body **21** and communicates with the internal cavity **22** through the passage **23.** The catheter tube **30** may be secured to the handle body **21** using a flanged connection, a fused connection, an adhesive, a press-fit connection, a threaded connection, or any other securing means. Alternatively, the catheter tube **30** may be secured to the handle body **21** using a connector or any other type of attachment device.

The illustrated catheter device **10** also includes an inner sleeve **40,** although such is not required. The inner sleeve **40** is a flexible, tubular member that is supported for sliding movement within the catheter tube **30,** the purpose of which will be explained below. The inner sleeve **40** can be made from any biocompatible material including, but not limited to, polyvinyl, polyethylene, nitinol, stainless steel, or a woven material. Further, the inner sleeve **40** can have any outer diameter, length, or wall thickness. The inner sleeve **40** need not be a tubular member but may alternatively be a solid wire, a braided wire, or the like.

A proximal end of the inner sleeve **40** extends from the catheter tube **30** and into the internal chamber **22** of the handle body **21.** The proximal end of the inner sleeve **40** is secured to the base portion **26** of the control member **25** for sliding movement therewith, the purpose of which will be explained below. The inner sleeve **40** can be secured to the base portion **26** by a flanged connection, a fused connection, an adhesive, a threaded connection, or any other securing means.

The inner sleeve **40** extends through an entire length of the catheter tube **30.** A distal end of the inner sleeve **40** that is opposite the handle assembly **20** is secured to the tip member **38,** which is in turn secured to the expandable portion **32.** The inner sleeve **40** may be secured to the tip member **38** in any manner including, but not limited to, a fused connection, an adhesive, a fastener, or the like.

Referring now to **FIGURE 1** through **FIGURE 7**, the operation of the catheter device **10** will now be described. Referring initially to **FIGURE 1** through **FIGURE 4**, the catheter device **10** is illustrated in a first operating mode. In the first operating mode, the control member **25** on the handle assembly **20** may be located in the forward position relative to the handle body **21.** The inner sleeve **40** fully extends into the catheter tube **30** such that the expandable portion **32** is in a closed position, as shown in **FIGURE 3** and **FIGURE 4**. In the closed position, the struts **34A** and **348** are generally parallel with one another and with the inner sleeve **40.** The slits **35A** and **358** (illustrated by the dashed lines in **FIGURE 3****)** remain in a generally closed configuration. As such, the expandable portion **32** defines an initial diameter **01,** which is generally the same diameter as the remaining length of the catheter tube **30.** The initial diameter **01** of the expandable portion **32** may, however, be any desired dimension.

Referring now to **FIGURE 5** through **FIGURE 7****,** the catheter device **10** is illustrated in a second operating mode. To achieve the second operating mode, the control member **25** may be moved from the forward position to the rearward position, as indicated by the direction arrow in **FIGURE 5****.** As the control member **25** is moved to the rearward position, the inner sleeve **40** is drawn within the catheter tube **30** thereby reducing the relative length of the inner sleeve **40** with respect to the catheter tube **30.** The distal end of the inner sleeve **40** is attached to the tip member **38,** as described above, causing the expandable portion **32** to become axially compressed between the tip member **38** and the distal end of the catheter tube **30.** As a result, the struts **34A** and **348** bow or expand outwardly in a generally arcuate fashion thereby defining the opened position. In the opened position, the expandable portion **32** defines a second diameter **02** that is larger than the initial diameter **01** when the expandable portion **32** is in the closed position. As shown in **FIGURE 6****,** the incising elements **36** are respectively positioned along the radially outer most surfaces of the struts **34A** and **348.** Further, the outer most surfaces of the struts **34A** and **348** may define a generally flat portion along a length thereof in the opened position, the purpose of which will be explained below, although such is not required. It should be appreciated that the struts **34A** and **348** can have any lengths such that the expandable portion **32** can achieve a desired overall second diameter **02** in the opened position. It should further be appreciated that the illustrated struts **34A** and **348** are merely exemplary, any number, size, and shape of struts are contemplated with any number of corresponding of longitudinally extending slits.

During operation of the catheter device **10,** the second diameter **02** can be increased or decreased by selective movement of the control member **25** between the forward and rearward positions. For example, a larger second diameter **02** can be achieved by moving the control member **25** further towards the rearward position. Conversely, a smaller second diameter **02** can be achieved by moving the control member **25** further towards the forward position. The visual indicator **24A** can be used to identify the instantaneous second diameter **02** of the expandable portion **32.** Alternatively (or in addition), the struts **34A** and **348** may be biased in the opened position so as to automatically expand outwardly to the second diameter **02** when the protective sheath **42** is slid back from the expandable portion **32.** As such, sliding movement of the protective sheath **42** relative to the struts **34A** and **348** can be used to selectively control the second diameter **02.** In this configuration, the inner sleeve **40** and the movable components of the handle assembly **20** may not be necessary.

When the catheter device **10** is in the second operating mode, the expandable portion **32** can be pulled along a guide wire **52** through the narrowed region of the blood vessel **50.** This may be accomplished by pulling on the handle assembly **20.** In doing so, the incising elements **36** may engage the atherosclerotic material **54** and create longitudinal incisions **56** therein. As shown in **FIGURE 6** and **FIGURE 7****,** the outer surface area of the arcuate shaped struts **34A** and **348,** which is adjacent to the incising elements **36,** is configured to ride along a surface of the atherosclerotic material **54,** thereby limiting the depth of the incisions **56** and preventing the incising elements **36** from cutting the walls of the blood vessel **50.** The expandable portion **32** can be moved any distance along the guide wire **52** to create incisions **56** having any desired length. After the incisions **56** are made in the atherosclerotic material **54,** the catheter device **10** can be returned to the first operating mode (shown in **FIGURE 1** through **FIGURE 4****)** by moving the control member **25** to the forward position. In doing so, the expandable portion **32** returns to the closed position.
The protective sheath **42** may be slid over the expandable portion **32** and the catheter tube **30** may be removed from the blood vessel **50.**

Alternatively, the catheter device **10** can be used to create additional incisions **56** in the atherosclerotic material **54.** For example, after the catheter device **10** has been returned to the first operating mode, the expandable portion **32** may be relocated within the narrowed region of the blood vessel **50.** The catheter tube **30** may then be rotated within the blood vessel **50** by rotating the handle assembly **20** so as to align the incising elements **36** with other portions of the atherosclerotic material **54.** The previous steps may then be repeated any number of times to make multiple passes through the narrowed region of the blood vessel **50** and create additional incisions in the atherosclerotic material **54.**

Thus, it should be appreciated that the illustrated catheter device **10** is advantageous in many respects. In one example, the second diameter **02** of the expandable portion **32** can be selectively controlled by operation of the handle assembly **20** or by sliding movement of the protective sheath **42.** This enables the catheter device **10** to be adapted for use in blood vessels **50** of different sizes or varying diameters. In another example, the illustrated catheter device **10** may apply varying magnitudes of radial forces to the atherosclerotic material **54** by controlling the amount of force being applied to the control member **25** on the handle assembly **20.** This enables the catheter device **10** to generate sufficient radial force to create incisions **56** in atherosclerotic material **54** while reducing the potential for tearing the walls of the blood vessel **50.** In yet another example, the catheter device **10** may be used to make any number of passes during a single procedure to make multiple incisions **56** in atherosclerotic material **54** of varying lengths and shapes.

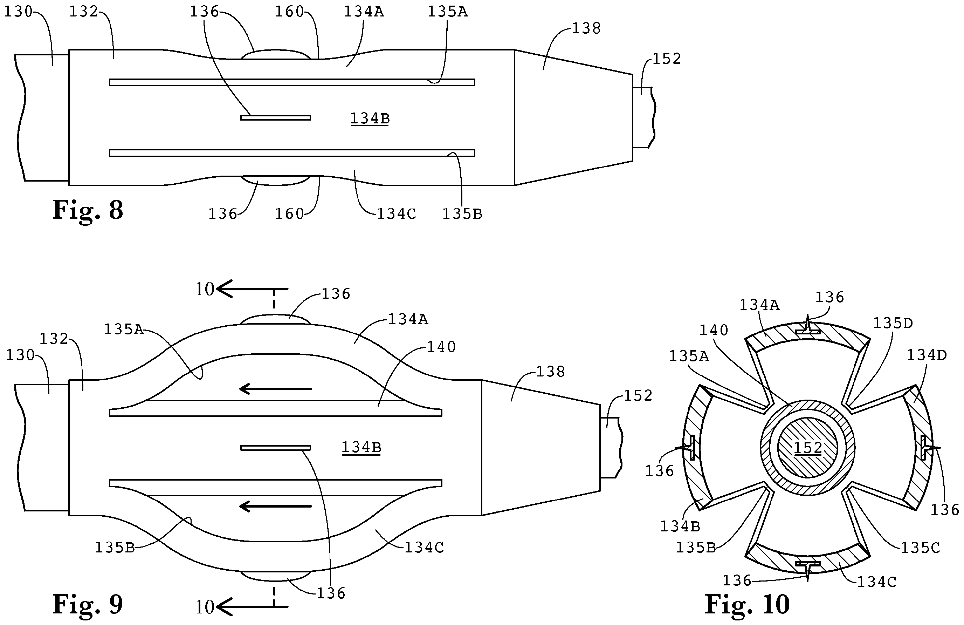

**FIGURE 8** through **FIGURE 10** illustrate a catheter tube **130** having an expandable portion **132** in accordance with another arrangement. The catheter tube **130** and the expandable portion **132** may include any structural features as described and illustrated above in the previous embodiment, although such is not required. Similar features have been numbered with common reference numerals but have been increased by **100** (i.e., **110, 120, 130,** etc.). It should be appreciated that similar features are structured similarly, operate similarly, and/or have the same function unless otherwise indicated by the drawings or this specification.

For example, the catheter tube **130** may extend from the handle assembly **20** along a guide wire **152** similar to previously described embodiments of the present invention. The expandable portion **132** is provided on a distal end of the catheter tube **130** and may include a tip member **138.** The catheter tube **130** may also include an inner sleeve **140** and a protective sheath **42,** which is also described above in the first embodiment.

In the illustrated embodiment, however, the expandable portion **132** includes four struts **134A, 1348, 134C,** and **1340** that are respectively separated by four longitudinally extending slits **135A, 1358, 135C,** and **1350.** The illustrated struts **134A, 1348, 134C,** and **1340** each include an incising element **136,** although such is not required. It should be appreciated that the expandable portion **132** may have any number or configuration of struts and incising elements as desired.

As shown in **FIGURE 8****,** the illustrated expandable portion **132** further includes recessed portions **160** that respectively extend into the outer surfaces of the struts **134A, 1348, 134C,** and **1340.** For example, the struts **134A, 1348, 134C,** and **1340** can be slightly bowed inwardly toward the inner sleeve **140** when in the closed position or, alternatively, may have a reduced thickness along a central portion thereof to create the recessed portions **160.** The illustrated incising elements **136** are respectively disposed within the recessed portions **160.** Thus, when the catheter tube **130** is inserted into a blood vessel, as described above, the recessed portions **160** help to prevent the incising elements **136** from coming into contact with inner walls of the blood vessel. On the other hand, when the expandable portion **132** is expanded to the opened position, as explained below, the incising elements **136** become exposed from the recessed portions **160.** It should be appreciated that the recessed portions **160** can eliminate or reduce the need for the protective sheath **42.**

The expandable portion **132** can be operated between the closed position (shown in **FIGURE 8**) and the opened position (shown in **FIGURE 9** and **FIGURE 10****)** by selective movement of the inner sleeve **140** relative to the catheter tube **130,** as described above in the first arrangement. Alternatively (or in addition), the struts **134A, 1348, 134C,** and **1340** may be biased in the opened position. In such an arrangement, the protective sheath **42** can be used to effect movement of the expandable portion **132** between the closed position and the opened position. It is notable that the number and shape of struts **134A, 1348, 134C** and **1340** are merely exemplary, any number and shape of struts and corresponding longitudinally extending slits is contemplated.

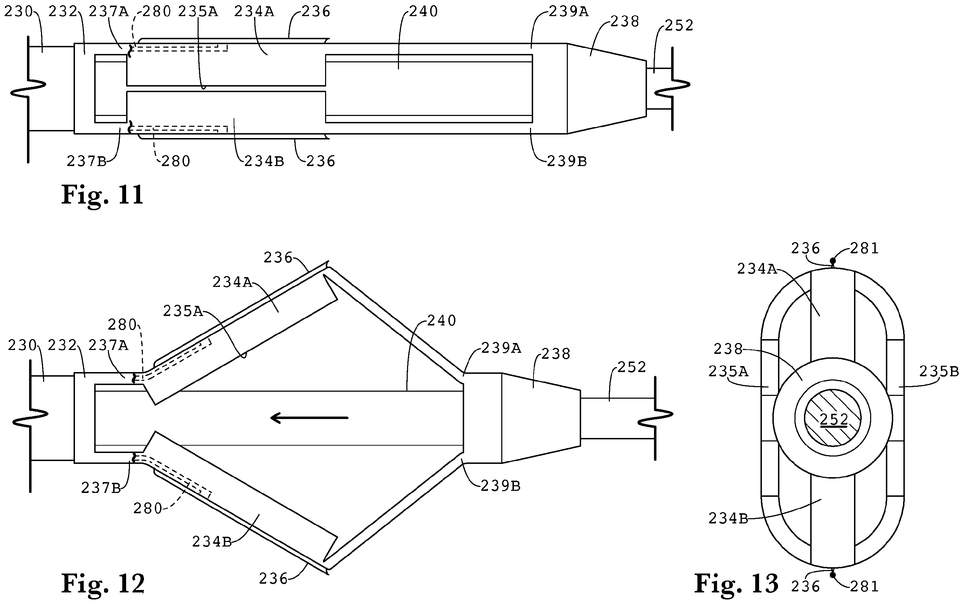

**FIGURE 11** through **FIGURE 13** illustrates a catheter tube **230** having an expandable portion **232,** in accordance with an exemplary embodiment of this invention. The catheter tube **230** and the expandable portion **232** may include any structural features as described and illustrated above in the previous arrangements, although such is not required. Similar features have been numbered with common reference numerals but have been increased by **200** (i.e., **210, 220, 230,** etc.). It should be appreciated that similar features are structured similarly, operate similarly, and/or have the same function unless otherwise indicated by the drawings or this specification.

For example, the catheter tube **230** may extend from the handle assembly **20** along a guide wire **252** similar to previously described arrangements. The expandable portion **232** is provided on a distal end of the catheter tube **230** and includes a pair of struts **234A** and **2348** that are separated by a pair of longitudinally extending slits **235A** and **2358.The** catheter tube **230** may also include a tip member **238,** an inner sleeve **240,** and a protective sheath **42,** which is described above in the first arrangement. It is notable that the number and shape of struts **134A, 1348, 134C** and **1340** are merely exemplary, any number, size, and shape of struts and corresponding longitudinally extending slits is contemplated.

Alternatively, the expandable portion **232** may include a first pair of weakened regions **237A, 2378** and a second pair of weakened regions **239A, 2398** that are respectively located at opposite ends of the struts **234A** and **2348.** The illustrated weakened regions **237A, 2378** and **239A, 2398** are formed by enlarged apertures that extend through side walls of the expandable portion **232** that function as hinges. The weakened regions **237A, 2378** and **239A, 2398** may help reduce the amount of bending stress in the side walls of the expandable portion **232** when the struts **234A** and **2348** are moved to the opened position. The struts **234A** and **2348** may include any number or configuration of weakened regions. Further, it should be appreciated that any of the other arrangements or embodiments in this disclosure may also include weakened regions similar to weakened regions **237A, 2378** and **239A, 2398.**

The illustrated struts **234A** and **2348** remain generally flat along respective lengths thereof in both the closed position (shown in **FIGURE 11**) and the opened position (shown in **FIGURE 12** and **FIGURE 13**) so as to form an apex, although such a configuration is not required. The incising elements **236** may be provided along the generally flat portion of the respective struts **234A** and **2348.** As such, the incising elements **236** may also function as stiffening members for increasing the strength of the struts **234A** and **2348.** Further, this configuration can reduce the amount of stress in the connection between the incising elements **236** and the struts **234A** and **2348,** which may otherwise be caused by bowing of the struts **234A** and **2348.**

**As** shown in **FIGURE 12**, end portions of the incising elements **236** may extend beyond the apex that is formed by each of the respective struts **234A** and **2348.** This configuration can increase the effective height of the incising elements **236** when the expandable portion **232** is in the opened position. As such, the incising elements **236** may have a reduced height when the expandable portion **232** is in the closed position, which may eliminate the need for the protective sheath **42.**

The expandable portion **232** can be operated between the closed position and the opened position by selective movement of the inner sleeve **240** relative to the catheter tube **230.** Alternatively (or in addition), the struts **234A** and **2348** can be biased in the opened position. In such an embodiment, the protective sheath **42** can be used to effect movement of the expandable portion **232** between the closed position and the opened position.

A mechanism is provided for delivering a drug to a base of one or more of the incising elements **236.** Specifically, the catheter device may comprise a drug delivery tube **280** having a first end that is located on or near
the handle assembly **20** and a second end that is located adjacent to the base of the one or more of the incising elements **236.** The drug delivery tube **280** allows a user of the catheter device to supply a drug or other material to the base of one or more of the incising elements **236** before, during, or after use thereof. The drug or other material can travel, by capillary action or otherwise, from the base of the incising element **236** to an adjacent portion of the blood vessel **50** or other desired area of interest.

The drug delivery tube **280** may be in communication with a control mechanism (not shown) located on the handle assembly **20 of** **FIGURE 1**. This control mechanism may allow the user of the catheter device to selectively introduce the amount and type of drug delivered to the incising element **236.** This control mechanism may include a signaling device (not shown) to communicate the type and amount of drug delivered, or other such information.

Additionally, as illustrated in **FIGURE 13** one or more of the incising elements may have a coating **281** of medicine provided thereon. The coating **281** allows a user of the catheter device to supply a drug or other material directly to the surface of one or more of the incising elements **236** before, during, or after use thereof. The drug or other material can travel (by capillary action or otherwise) from the coating **281** on the incising element **236** to an adjacent portion of the blood vessel **50** or other desired area of interest.

The coating **281** may be used in conjunction with a protective sheath, such as protective sheath **42 of** **FIGURE 1**. The protective sheath **42** may cover the device, including the incising element **236,** prior to the device being located at the site for treatment. This may prevent the coating **281** from being inadvertently removed by contact with the blood vessel wall **50** at an unintended area. Further, said protective sheath **42** may form a watertight seal over the expandable portion **232.** The watertight seal may prevent the coating **281** from being exposed to blood vessel fluids before being located at the treatment site, thereby preventing dilution and loss of the coating **281.**

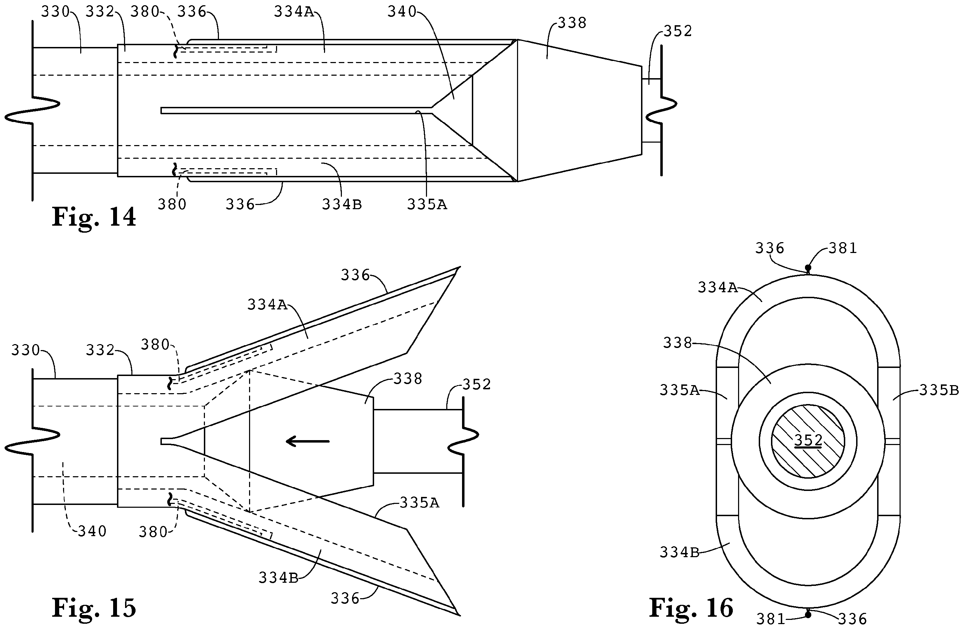

**FIGURE 14** though **FIGURE 16** illustrates a catheter tube **330** having an expandable portion **332,** in accordance with another exemplary embodiment of this invention. The catheter tube **330** and the expandable portion **332** may include any structural features as described and illustrated above in the previous arrangements and embodiment, although such is not required. Similar features have been numbered with common reference numerals but have been increased by **300** (i.e., **310, 320, 330,** etc.). It should be appreciated that similar features are structured similarly, operate similarly, and/or have the same function unless otherwise indicated by the drawings or this specification.

For example, the catheter tube **330** may extend from the handle assembly **20** along a guide wire **352** similar to previously described embodiments of the present invention. The expandable portion **332** is provided on a distal end of the catheter tube **330** and may include a tip member **338.** The catheter tube **330** may also include an inner sleeve **340** that is attached to the tip member **338** and a protective sheath **42,** which is also described above.

In the illustrated embodiment, however, the expandable portion **332** includes a pair of struts **334A** and **3348** that are supported thereon in a cantilevered manner (i.e., not attached to one another or to the tip member **338** at their distal ends), the purpose of which will be explained below. The struts **334A** and **3348** are separated by a pair of longitudinally extending slits **335A** and **3358** that extend from the end of the expandable portion **332.** A pair of incising elements **336** is respectively provided along outer surfaces of the struts **334A** and **3348.** It should be appreciated, however, that the expandable portion **332** may have any number or configuration of struts, corresponding longitudinally extending slits, and incising elements as desired.

As shown in **FIGURE 15** and **FIGURE 16****,** the illustrated struts **334A** and **3348** are supported on the expandable portion **332** so that they can be splayed open in a Y shaped configuration. For example, the struts **334A** and **3348** may be splayed open by drawing the inner sleeve **340** within the catheter tube **330,** as described above in other embodiments. In doing so, the tip member **338** slides along the inner surfaces of the struts **334A** and **3348** and pivots them outwardly. Alternatively (or in addition), the struts **334A** and **3348** may be biased in the splayed open position. In such an embodiment, the protective sheath **42** can be used to effect movement of the expandable portion **332** between the closed position and the splayed open position.

The struts **334A** and **3348** remain generally flat along their respective lengths in both the closed position (shown in **FIGURE 14**) and the splayed open position (shown in **FIGURE 15**), although such is not required. As such, the incising elements **336** may also function as stiffening members for increasing the strength of the struts **334A** and **3348.** Further, this configuration can reduce the amount of stress in the connection between the incising elements **336** and the struts **334A** and **3348,** which may otherwise be caused by bowing of the struts **334A** and **3348.**

**As** shown in **FIGURE 15****,** end portions of the incising elements **336** may extend beyond the distal ends of the respective struts **334A** and **3348.** This configuration can increase the effective height of the incising elements **336** when the expandable portion **332** is in the splayed open position. As such, the incising elements **336** may have a reduced height when the expandable portion **332** is in the closed position, which may eliminate the need for the protective sheath **42.**

A mechanism may be further provided for delivering a drug to a base of one or more of the incising elements **336.** Specifically, the catheter device may comprise a drug delivery tube **380** having a first end that is located on or near the handle assembly and a second end that is located adjacent to the base of the one or more of the incising elements **336.** The drug delivery tube **380** allows a user of the catheter device to supply a drug or other material to the base of one or more of the incising elements **336** before, during, or after use thereof. The drug or other material can travel, by capillary action or otherwise, from the base of the incising element **336** to an adjacent portion of the blood vessel **50** or other desired area of interest.

The drug delivery tube **380** may be in communication with a control mechanism (not shown) located on the handle assembly **20 of** **FIGURE 1****.** This control mechanism may allow the user of the catheter device to selectively introduce the amount and type of drug delivered to the incising element **336.** This control mechanism may include a signaling device (not shown) to communicate the type and amount of drug delivered, or other such information.

Additionally, as illustrated in **FIGURE 16** one or more of the incising elements may have a coating **381** of medicine provided thereon. The coating **381** allows a user of the catheter device to supply a drug or other material directly to the surface of one or more of the incising elements **336** before, during, or after use thereof. The drug or other material can travel (by capillary action or otherwise) from the coating **381** on the incising element **336** to an adjacent portion of the blood vessel **50** or other desired area of interest.

The coating **381** may be used in conjunction with a protective sheath, such as protective sheath **42 of** **FIGURE 1**. The protective sheath may cover the device, including the incising element **336,** prior to the device being located at the site for treatment. This may prevent the coating **381** from being inadvertently removed by contact with the blood vessel wall at an unintended area. Further, said protective sheath may form a watertight seal over the expandable portion. The watertight seal may prevent the coating **381** from being exposed to blood vessel fluids before being located at the treatment site, thereby preventing dilution and loss of the coating **381.**

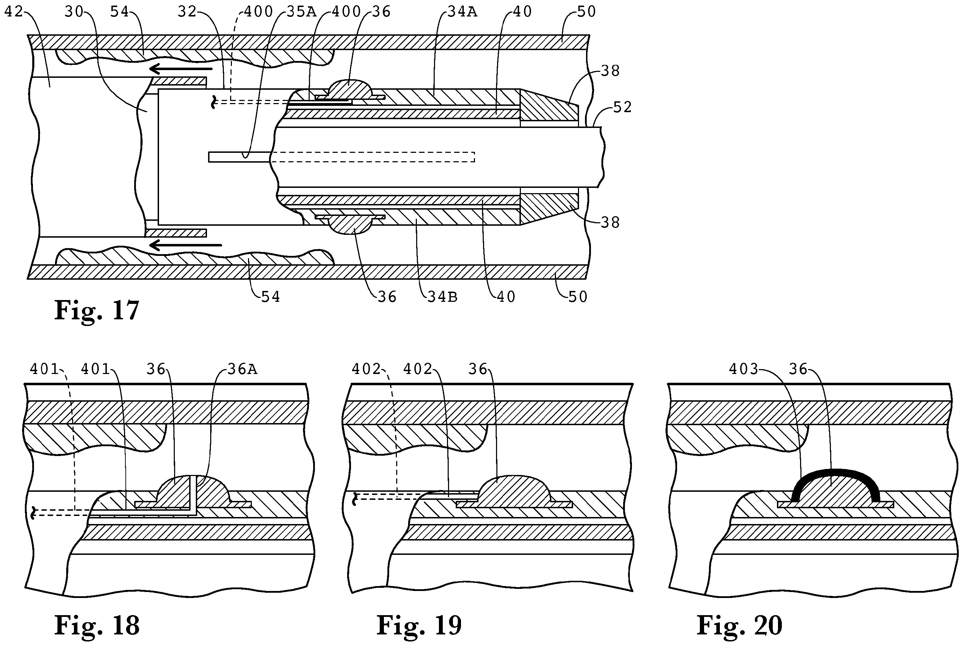

Additionally, any of the other delivery mechanisms discussed and illustrated in **FIGURE 17** through **FIGURE 20** may be used with the embodiments illustrated and discussed in **FIGURE 14** though **FIGURE 16**.

**FIGURE 17** is an enlarged cross-sectional side view similar to **FIGURE 3** illustrating another exemplary embodiment of this invention, wherein a mechanism is provided for delivering a drug to a base of one or more of the incising elements **36.** Specifically, the catheter device **10** may comprise a drug delivery tube **400** having a first end that is located on or near the handle assembly **20** and a second end that is located adjacent to the base of the one or more of the incising elements **36.** The drug delivery tube **400** allows a user of the catheter device **10** to supply a drug or other material to the base of one or more of the incising elements **36** before, during, or after use thereof. The drug or other material can travel, by capillary action or otherwise, from the base of the incising element **36** to an adjacent portion of the blood vessel **50** or other desired area of interest.

The drug delivery tube **400** may be in communication with a control mechanism (not shown) located on the handle assembly **20 of** **FIGURE 1****.** This control mechanism may allow the user of the catheter device **10** to selectively introduce the amount and type of drug delivered to the incising element **36.** This control mechanism may include a signaling device (not shown) to communicate the type and amount of drug delivered, or other such information.

**FIGURE 18** is an enlarged cross-sectional side view similar to **FIGURE 17** illustrating another exemplary embodiment of this invention, wherein a mechanism is provided for delivering a drug to a surface of one or more of the incising elements **36.** Specifically, the catheter device **10** may comprise a drug delivery tube **401** having a first end that is located on or near the handle assembly **20** and a second end that is located adjacent to the base of the one or more of the incising elements **36.** The incising element **36** may further comprise an internal passageway **36a** that extends from the base thereof to a surface thereof. The drug delivery tube **401** and the passageway **36a** allow a user of the catheter device **10** to supply a drug or other material to the surface of one or more of the incising elements **36** before, during, or after use thereof. The drug or other material can travel through both the drug delivery tube **401** and the passageway **36a** to an adjacent portion of the blood vessel **50** or other desired area of interest.

The drug delivery tube **401** and internal passageway **36a** may be in communication with a control mechanism (not shown) located on the handle assembly **20 of** **FIGURE 1**. This control mechanism may allow the user of the catheter device **10** to selectively introduce the amount and type of drug delivered to the surface of the incising element **36.** This control mechanism may include a signaling device (not shown) to communicate the type and amount of drug delivered, or other such information.

**FIGURE 19** is an enlarged cross-sectional side view similar to **FIGURE 17** illustrating another arrangement, wherein a mechanism is provided for delivering a drug to a surface of one or more of the incising elements **36.** Specifically, the catheter device **10** may comprise a drug delivery tube **402** having a first end that is located on or near the handle assembly **20** and a second end that is located adjacent to the surface of the one or more of the incising elements **36.** The drug delivery tube **402** allows a user of the catheter device **10** to supply a drug or other material directly to the surface of one or more of the incising elements **36** before, during, or after use thereof. The drug or other material may travel, by capillary action or otherwise, from the surface of the incising element **36** to an adjacent portion of the blood vessel **50** or other desired area of interest.

The drug delivery tube **402** may be in communication with a control mechanism (not shown) located on the handle assembly **20 of** **FIGURE 1**. This control mechanism may allow the user of the catheter device **10** to selectively introduce the amount and type of drug delivered to the surface of the incising element **36.** This control mechanism may include a signaling device (not shown) to communicate the type and amount of drug delivered, or other such information.

**FIGURE 20** is an enlarged cross-sectional side view similar to **FIGURE 17** illustrating another arrangement, wherein one of the incising elements has a coating **403** of medicine provided thereon. The coating **403** allows a user of the catheter device **10** to supply a drug or other material directly to the surface of one or more of the incising elements **36** before, during,
or after use thereof. The drug or other material can travel (by capillary action or otherwise) from the coating **403** on the incising element **36** to an adjacent portion of the blood vessel **50** or other desired area of interest.

The coating **403** may be used in conjunction with a protective sheath, such as protective sheath **42 of** **FIGURE 1**. The protective sheath may cover the device, including the incising element **36,** prior to the device being located at the site for treatment. This may prevent the coating **403** from being inadvertently removed by contact with the blood vessel wall at an unintended area. Further, said protective sheath may form a watertight seal over the expandable portion. The watertight seal may prevent the coating **403** from being exposed to blood vessel fluids before being located at the treatment site, thereby preventing dilution and loss of the coating **403.**

Having shown and described a preferred embodiment of the invention, those skilled in the art will realize that many variations and modifications may be made to affect the described invention and still be within the scope of the claimed invention. It is the intention, therefore, to limit the invention only as indicated by the scope of the claims.

## Claims

1. An intravascular catheter device (10) comprising:
a handle assembly (20);
a catheter tube (30, 130, 230, 330) extending from the handle assembly (20) and including a selectively expandable portion (32) having a plurality of struts (34A, 34B, 134A, 134B, 134C, 134D, 234A, 234B, 334A, 334B) each defining an outer surface, the expandable portion (32) being operable between a closed position, wherein the expandable portion (32) has a first diameter, and an opened position, wherein the expandable portion (32) has a second diameter that is larger than the first diameter;
an incising element (36, 136, 236, 336) provided on the outer surface of at least one of the struts (34A, 34B, 134A, 134B, 134C, 134D, 234A, 234B, 334A, 334B), the incising element (36, 136, 236, 336) having a sharpened edge that extends outwardly in a radial direction from the outer surface of the strut (34A, 34B, 134A, 134B, 134C, 134D, 234A, 234B, 334A, 334B) and extends parallel with a longitudinal axis of the expandable portion (32) for creating an incision in atherosclerotic material (54) located within a blood vessel (50) when the expandable portion (32) is in the opened position and moved axially through the blood vessel (50);
a drug delivery device comprising a drug delivery tube (280, 400, 401, 402) which extends along the catheter tube (30, 130, 230, 330) and through the at least one of the struts (34A, 34B, 134A, 134B, 134C, 134D, 234A, 234B, 334A, 334B), is in communication with the handle assembly (20), and is configured to permit the delivery of a drug to the base of the incising element (36, 136, 236, 336).

2. The intravascular catheter device (10) of Claim 1, wherein:
the struts (34A, 34B, 134A, 134B, 134C, 134D, 234A, 234B, 334A, 334B) are supported in a cantilevered fashion on the expandable portion (32) so as to be splayed open when in the opened position; and
further comprising a tip member (38) that is supported for sliding movement along the inner surfaces of the struts (34A, 34B, 134A, 134B, 134C, 134D, 234A, 234B, 334A, 334B) so as to pivot the struts (34A, 34B, 134A, 134B, 134C, 134D, 234A, 234B, 334A, 334B) between the closed position and the opened position.

3. The intravascular catheter device (10) of Claim 1, wherein:
the drug delivery tube (280, 400, 401, 402) and the incising element (36, 136, 236, 336) are configured to deliver the drug to the surface of the incising element (36, 136, 236, 336) by capillary action.

4. The intravascular catheter device (10) of Claim 1, wherein:
the incising element (36, 136, 236, 336) further comprises an internal passageway (36a) extending from the drug delivery tube (280, 400, 401, 402) to the incising element (36, 136, 236, 336).

5. The intravascular catheter device (10) of Claim 4, wherein:
the internal passageway (36a) extends from the drug delivery tube (280, 400, 401, 402) to the surface of the incising element (36, 136, 236, 336) and is configured to permit the delivery of the drug to the surface of the incising element (36, 136, 236, 336).

6. An intravascular catheter device (10) of Claim 1, wherein:
the drug delivery tube (280, 400, 401, 402) terminates adjacent to the base of the incising element (36, 136, 236, 336).

7. The intravascular catheter device (10) of Claim 6, wherein:
the drug delivery tube (280, 400, 401, 402) and the incising element (36, 136, 236, 336) are configured to deliver the drug to the surface of the incising element (36, 136, 236, 336) by capillary action.

8. The intravascular catheter device (10) of Claim 1, wherein:
the handle assembly (20) further comprises an indicator (24A) that identifies the amount of the drug which has been released.

9. The intravascular catheter device (10) of Claim 1:
further comprising a protective sheath (42) that is supported for sliding movement along an outer surface of the catheter tube (30, 130, 230, 330); and wherein the protective sheath (42) is configured to selectively surround the expandable portion (32).

10. The intravascular catheter device (10) of Claim 9:
further comprising a drug coating (281, 381) located on the outer surface of at one of the incising elements (36, 136, 236, 336); and
wherein the protective sheath (42) is configured to protect the drug coating (281,381) from being inadvertently removed by contact with an outside object.

11. The intravascular catheter device (10) of Claim 10 wherein:
the protective sheath (42) forms a watertight seal around at least the incising element (36, 136, 236, 336) with the drug coating (281, 381).

12. The intravascular catheter device (10) of claim 1 further comprising:
an inner sleeve (40) that is supported for sliding movement within the catheter tube (30, 130, 230, 330), the inner sleeve (40) is connected between the control member on the handle assembly (20) and a distal end of the expandable portion (32) for selectively controlling movement of the expandable portion (32) between the closed position and the opened position.

## Patentansprüche

1. Intravaskuläre Kathetervorrichtung (10), umfassend:
eine Griffanordnung (20);
einen Katheterschlauch (30, 130, 230, 330), der sich von der Griffanordnung (20) erstreckt und einen selektiv expandierbaren Abschnitt (32) einschließt, der mehrere Streben (34A, 34B, 134A, 134B, 134C, 134D, 234A, 234B, 334A, 334B) aufweist, von denen jede eine Außenfläche definiert, wobei der expandierbare Abschnitt (32) zwischen einer geschlossenen Position, in welcher der expandierbare Abschnitt (32) einen ersten Durchmesser aufweist, und einer offenen Position, in welcher der expandierbare Abschnitt (32) einen zweiten Durchmesser aufweist, der größer ist als der erste Durchmesser, betreibbar ist;
ein Einschneideelement (36, 136, 236, 336), das an der Außenfläche von mindestens einer der Streben (34A, 34B, 134A, 134B, 134C, 134D, 234A, 234B, 334A, 334B) vorgesehen ist, wobei das Einschneideelement (36, 136, 236, 336) eine scharfe Kante aufweist, die sich in radialer Richtung von der Außenfläche der Strebe (34A, 34B, 134A, 134B, 134C, 134D, 234A, 234B, 334A, 334B) nach außen erstreckt und sich parallel entlang einer Längsachse des expandierbaren Abschnitts (32) erstreckt, um in atherosklerotischem Material (54) innerhalb eines Blutgefäßes (50) einen Einschnitt zu erzeugen, wenn der expandierbare Abschnitt (32) sich in der geöffneten Position befindet und axial durch das Blutgefäß (50) bewegt wird;
eine Arzneimittelabgabevorrichtung, umfassend einen Arzneimittelabgabeschlauch (280, 400, 401, 402), der sich entlang des Katheterschlauchs (30, 130, 230, 330) und durch die mindestens eine der Streben (34A, 34B, 134A, 134B, 134C, 134D, 234A, 234B, 334A, 334B) erstreckt, der mit der Griffanordnung (20) in Verbindung steht und dazu ausgelegt ist, die Abgabe eines Arzneimittels an die Basis des Einschneideelements (36, 136, 236, 336) zu ermöglichen.

2. Intravaskuläre Kathetervorrichtung (10) nach Anspruch 1, wobei:
die Streben (34A, 34B, 134A, 134B, 134C, 134D, 234A, 234B, 334A, 334B) auf freitragende Weise an dem expandierbaren Abschnitt (32) gehalten werden, um in der geöffneten Position gespreizt zu werden; und
weiterhin umfassend ein Spitzenelement (38), das gehalten wird, um entlang der Innenflächen der Streben (34A, 34B, 134A, 134B, 134C, 134D, 234A, 234B, 334A, 334B) zu gleiten, um die Streben (34A, 34B, 134A, 134B, 134C, 134D, 234A, 234B, 334A, 334B) zwischen der geschlossenen Position und der offenen Position zu schwenken.

3. Intravaskuläre Kathetervorrichtung (10) nach Anspruch 1, wobei:
der Arzneimittelabgabeschlauch (280, 400, 401, 402) und das Einschneideelement (36, 136, 236, 336) dazu ausgelegt sind, das Arzneimittel mittels Kapillarwirkung zu der Oberfläche des Einschneideelements (36, 136, 236, 336) zu transportieren.

4. Intravaskuläre Kathetervorrichtung (10) nach Anspruch 1, wobei:
das Einschneideelement (36, 136, 236, 336) weiterhin einen inneren Durchgang (36a) umfasst, der sich von dem Arzneimittelabgabeschlauch (280, 400, 401, 402) zu dem Einschneideelement (36, 136, 236, 336) erstreckt.

5. Intravaskuläre Kathetervorrichtung (10) nach Anspruch 4, wobei:
sich der innere Durchgang (36a) von dem Arzneimittelabgabeschlauch (280, 400, 401, 402) zu der Oberfläche des Einschneideelements (36, 136, 236, 336) erstreckt und dazu ausgelegt ist, die Abgabe des Arzneimittels an die Oberfläche des Einschneideelements (36, 136, 236, 336) zu ermöglichen.

6. Intravaskuläre Kathetervorrichtung (10) nach Anspruch 1, wobei:
der Arzneimittelabgabeschlauch (280, 400, 401, 402) neben der Basis des Einschneideelements (36, 136, 236, 336) endet.

7. Intravaskuläre Kathetervorrichtung (10) nach Anspruch 6, wobei:
der Arzneimittelabgabeschlauch (280, 400, 401, 402) und das Einschneideelement (36, 136, 236, 336) dazu ausgelegt sind, das Arzneimittel mittels Kapillarwirkung zu der Oberfläche des Einschneideelements (36, 136, 236, 336) zu transportieren.

8. Intravaskuläre Kathetervorrichtung (10) nach Anspruch 1, wobei:
die Griffanordnung (20) weiterhin ein Anzeigegerät (24A) umfasst, das die Menge des freigesetzten Arzneimittels identifiziert.

9. Intravaskuläre Kathetervorrichtung (10) nach Anspruch 1:
weiterhin umfassend eine Schutzhülle (42), die zur Gleitbewegung entlang einer Außenfläche des Katheterschlauchs (30, 130, 230, 330) gehalten wird; und wobei die Schutzhülle (42) dazu ausgelegt ist, den expandierbaren Abschnitt (32) selektiv zu umgeben.

10. Intravaskuläre Kathetervorrichtung (10) nach Anspruch 9:
weiterhin umfassend eine Arzneimittelbeschichtung (281, 381), die sich an der Außenfläche eines der Einschneideelemente (36, 136, 236, 336) befindet; und
wobei die Schutzhülle (42) dazu ausgelegt ist, die Arzneimittelbeschichtung (281, 381) davor zu schützen, durch Kontakt mit einem externen Objekt unbeabsichtigt entfernt zu werden.

11. Intravaskuläre Kathetervorrichtung (10) nach Anspruch 10 wobei:
die Schutzhülle (42) mit der Arzneimittelbeschichtung (281, 381) einen wasserdichten Verschluss um mindestens das Einschneideelement (36, 136, 236, 336) bildet.

12. Intravaskuläre Kathetervorrichtung (10) nach Anspruch 1, weiterhin umfassend:
eine Innenhülse (40), die für eine Gleitbewegung innerhalb des Katheterschlauchs (30, 130, 230, 330) gehalten wird, wobei die Innenhülse (40) mit dem Steuerelement an der Griffanordnung (20) und einem distalen Ende des expandierbaren Abschnitts (32) verbunden ist, um die Bewegung des expandierbaren Abschnitts (32) zwischen der geschlossenen Position und der offenen Position selektiv zu steuern.

## Revendications

1. Un dispositif de cathéter intravasculaire (10) comprenant :
un ensemble poignée (20) ;
un tube de cathéter (30, 130, 230, 330) s'étendant à partir de l'ensemble poignée (20) et comprenant une partie sélectivement extensible (32) ayant une pluralité d'entretoises (34A, 34B, 134A, 134B, 134C, 134D, 234A, 234B, 334A, 334B) chacune définissant une surface extérieure, la partie extensible (32) pouvant être actionnée entre une position fermée, dans lequel la partie extensible (32) a un premier diamètre, et une position ouverte, dans lequel la partie extensible (32) a un deuxième diamètre qui est plus grand que le premier diamètre ;
un élément d'incision (36, 136, 236, 336) prévu sur la surface extérieure d'au moins une des entretoises (34A, 34B, 134A, 134B, 134C, 134D, 234A, 234B, 334A, 334B), l'élément d'incision (36, 136, 236, 336) ayant un bord aiguisé qui s'étend vers l'extérieur dans une direction radiale à partir de la surface extérieure de l'entretoise (34A, 34B, 134A, 134B, 134C, 134D, 234A, 234B, 334A, 334B) et s'étend parallèlement à un axe longitudinal de la partie extensible (32) pour créer une incision dans une matière athéroscléreuse (54) située dans un vaisseau sanguin (50) lorsque la partie extensible (32) est dans la position ouverte et déplacée axialement à travers le vaisseau sanguin (50) ; un dispositif d'administration de médicament comprenant un tube d'administration de médicament (280, 400, 401, 402) qui s'étend le long du tube de cathéter (30, 130, 230, 330) et à travers l'au moins une des entretoises (34A, 34B, 134A, 134B, 134C, 134D, 234A, 234B, 334A, 334B), est en communication avec l'ensemble poignée (20), et est configuré pour permettre l'administration d'un médicament à la base de l'élément d'incision (36, 136, 236, 336).

2. Le dispositif de cathéter intravasculaire (10) selon la revendication 1, dans lequel :
les entretoises (34A, 34B, 134A, 134B, 134C, 134D, 234A, 234B, 334A, 334B) sont supportées en porte-à-faux sur la partie extensible (32) de manière à être évasées en position ouverte ; et
comprenant en outre un élément de pointe (38) qui est supporté pour un mouvement coulissant le long des surfaces intérieures des entretoises (34A, 34B, 134A, 134B, 134C, 134D, 234A, 234B, 334A, 334B) de manière à faire pivoter les entretoises (34A, 34B, 134A, 134B, 134C, 134D, 234A, 234B, 334A, 334B) entre la position fermée et la position ouverte.

3. Le dispositif de cathéter intravasculaire (10) selon la revendication 1, dans lequel :
le tube d'administration de médicament (280, 400, 401, 402) et l'élément d'incision (36, 136, 236, 336) sont configurés pour administrer le médicament à la surface de l'élément d'incision (36, 136, 236, 336) par action capillaire.

4. Le dispositif de cathéter intravasculaire (10) selon la revendication 1, dans lequel :
l'élément d'incision (36, 136, 236, 336) comprend en outre un passage interne (36a) s'étendant à partir du tube d'administration de médicament (280, 400, 401, 402) jusqu'à l'élément d'incision (36, 136, 236, 336).

5. Le dispositif de cathéter intravasculaire (10) selon la revendication 4, dans lequel :
le passage interne (36a) s'étend à partir du tube d'administration de médicament (280, 400, 401, 402) jusqu'à la surface de l'élément d'incision (36, 136, 236, 336) et est configuré pour permettre l'administration du médicament à la surface de l'élément d'incision (36, 136, 236, 336).

6. Un dispositif de cathéter intravasculaire (10) selon la revendication 1, dans lequel :
le tube d'administration de médicament (280, 400, 401, 402) se termine de manière adjacente à la base de l'élément d'incision (36, 136, 236, 336).

7. Le dispositif de cathéter intravasculaire (10) selon la revendication 6, dans lequel :
le tube d'administration de médicament (280, 400, 401, 402) et l'élément d'incision (36, 136, 236, 336) sont configurés pour administrer le médicament à la surface de l'élément d'incision (36, 136, 236, 336) par action capillaire.

8. Le dispositif de cathéter intravasculaire (10) selon la revendication 1, dans lequel :
l'ensemble poignée (20) comprend en outre un indicateur (24A) qui identifie la quantité de médicament qui a été libérée.

9. Le dispositif de cathéter intravasculaire (10) selon la revendication 1 :
comprenant en outre une gaine de protection (42) qui est supportée pour un mouvement coulissant le long d'une surface extérieure du tube de cathéter (30, 130, 230, 330) ; et
dans lequel la gaine de protection (42) est configurée pour entourer sélectivement la partie extensible (32).

10. Le dispositif de cathéter intravasculaire (10) selon la revendication 9 :
comprenant en outre un enrobage de médicament (281, 381) situé sur la surface extérieure de l'un des éléments d'incision (36, 136, 236, 336) ; et
dans lequel la gaine de protection (42) est configurée pour protéger l'enrobage de médicament (281, 381) contre son retrait accidentel par contact avec un objet extérieur.

11. Le dispositif de cathéter intravasculaire (10) selon la revendication 10 dans lequel :
la gaine de protection (42) forme un joint d'étanchéité hermétique vis-à-vis de l'eau autour au moins de l'élément d'incision (36, 136, 236, 336) avec l'enrobage de médicament (281, 381).

12. Le dispositif de cathéter intravasculaire (10) selon la revendication 1 comprenant en outre :
un manchon intérieur (40) qui est supporté pour un mouvement coulissant à l'intérieur du tube de cathéter (30, 130, 230, 330), le manchon intérieur (40) est relié entre l'élément de contrôle sur l'ensemble poignée (20) et une extrémité distale de la partie extensible (32) pour contrôler sélectivement le mouvement de la partie extensible (32) entre la position fermée et la position ouverte.
